# EUROPEAN PATENT APPLICATION

(11) **EP 2 740 498 A2**
(43) Date of publication of application: **11.06.2014**
(21) Application number: 13184537.2
(22) Date of filing: 02.07.2009
(51) Int. Cl.: A61L 27/18, A61L 27/38, A61L 27/58, A61L 26/00, A61L 27/20, A61L 27/24, A61L 27/26, A61L 27/50, A61L 27/48, A61L 27/52

(54) **Compositions and methods for tissue filling and regeneration**

(30) Priority: 02.07.2008 US 77683 P
(62) Divisional of application: 09774553.3
(71) Applicant: Allergan, Inc., Irvine, CA 92612 (US)
(72) Inventor: Van Epps, Dennis E., Goleta, California 93117 (US)
(74) Representative: HOFFMANN EITLE

(57) **Abstract**

Injectable compositions are provided, which include both a living cellular component and a filler component conducive to cell growth. The compositions are capable of providing both immediate tissue filling and long term tissue regeneration.

## Description

### Related Application

This application claims the benefit of United States Provisional Patent Application No. 61/077,683, filed on July 2, 2008, the entire disclosure of which is incorporated herein by this reference.

### Background

The present invention generally relates to tissue filling, augmentation and regeneration, and more specifically provides combined cell and filler compositions for augmenting, replacing, cosmetically enhancing and/or repairing soft tissue in mammals.

Various products have been injected into the human body to augment soft tissue and correct skin defects. Early examples of such products include paraffin, petrolatum, vegetable oils, lanolin, bees wax, and silicone.

Bovine and human collagen have gained widespread use as injectable materials for soft tissue augmentation and filling. Collagen, the principal extracellular structural protein of the animal body, has been used as an implant material to replace or augment connective tissue, such as skin, tendon, cartilage and bone. Additionally, collagen has been injected or implanted into the human body for cosmetic purposes for a number of years.

Hyaluronic acid (HA) is a glycosaminoglycan that is naturally found in the human body and is widely distributed throughout connective, epithelial, and neural tissues. In 2003, the Food and Drug Administration approved HA-based injections for correcting facial wrinkles.

Schroeder et al., U.S. Patent Application Serial No. No.12/247,175, filed October 7, 2008, describes crosslinked hyaluronic acid and collagen, for augmenting soft tissue in mammals and methods for preparing same. The entire disclosure of this publication is incorporated herein by this reference.

### Summary of the Invention

The present invention generally provides improved compositions for augmenting and repairing the skin and soft tissues for cosmetic and therapeutic purposes. The present compositions, and methods for making and using such compositions, are useful for filling, augmentation and/or regeneration of soft tissues in a mammalian patient, for example a human being.

In a broad aspect of the invention, the compositions are injectable compositions which include both a living cellular component and a filler component conducive to maintaining viability of cell growth, for example, maintaining viability of cells of the cellular component. The compositions are capable of providing both immediate tissue filling and long term tissue regeneration.

In accordance with one aspect of the invention, the cellular component of the present compositions comprises cells selected from the group of cells consisting of stem cell in-situ culture, progenitor cells, adipose cells, adipose-derived stem cells, mcscnchcmal stem cells, endothelial cell precursors, proliferating cells, differentiation-committed cells, and regenerative cells and stem cell lines.

In one embodiment, the filler component is selected from the group consisting of hyaluronic acid, chitosan, biodegradable polymers, collagen, crosslinked hyaluronic acid/collagen, hydrogels, derivatives and combinations thereof. In a more specific embodiment, the filler component comprises a hydrogels that undergo sol-gel transformation via thermal, ionic, hydrophobic, pH, or catalyst induction.

In one aspect, the present invention provides methods for preparing an injectable or implantable filling and regenerating composition, wherein the composition includes both a cellular component and a filler component. The method may include the step of combining, for example, mixing, human stem cells with a filler component, for example, a hyaluronic acid-based filler material or premixing the cellular component with a soluble form of the material prior to gelation. Addition of integrin binding components provide adhesion and signals to retain viability or enhance cell growth, Such components include one or more of laminin, vitronectin, fibronectin, elastin and peptides and analogs thereof.

In yet another aspect of the invention, injectable or implantable compositions are provided which are useful for tissue filling and regeneration, the compositions including a cellular component and a filler component effective to support proliferation of cells or tissue growth for both and immediate and sustained period of time, for example, for greater than six months.

In a further aspect of the invention, a kit for use in filling and regenerating tissue is provided. The kit may comprise a cannula, syringe, a cellular and/or filler component and instructions for use. The syringe includes a composition in accordance with the invention, for example, a composition comprising a cellular component and a filler component or a two compartment system which maintains separation of the cellular and filler component until or near the time of injection.

In other aspects of the invention, the compositions arc useful as a component of a kit for use in filling and regenerating tissue. Such a kit may comprise a filler material, a tool for extracting autologous cells from a patient, and an injector device for cnabling injection of a mixture of the filler component and extracted autologous cells into a target region of the patient. In other aspects of the invention, the compositions are in the form of a topical wound covering for filling and regeneration.

Each and every feature described herein, and each and every combination of two or more of such features, is included within the scope of the present invention provided that the features included in such a combination arc not muhially inconsistent.

### Brief Description of the Drawings

Fig. 1 is a schematic view of a kit in accordance with one embodiment of the invention.
Fig. 2 is a schematic view of another kit in accordance with an embodiment of the invention.

### Detailed Description

Accordingly, the present invention provides methods for producing highly useful, safe and effective compositions for soft tissue augmentation, replacement, cosmetic enhancement and/or tissue repair in a mammal, for example, a human being. The invention further provides products, for example, injectable compositions, useful for substantially immediate tissue filling or volumizing of tissue as well as support for long term tissue regeneration.

The present invention provides methods for breast augmentation and reconstruction where a combination of volume and tissue regeneration may be desired. Similarly, filing of any tissue void either natural or created by surgical procedure for removal of tissue, corticosteroid treatment, immunologic reaction resulting in lipoatrophy, tissue damage resulting from impact injuries, radio or chemical or drug treatment where there is a desire to both fill and regenerate tissue at a particular site. Similarly, the present compositions can be used as a means for reducing scar tissue as a result of the active vascularization and dissolution of scar tissue either as a preventative or post treatment procedure based on the delivery of viable fat-derived cells or stem or progenitor cells to the site.

In one embodiment, a composition in accordance with the invention generally comprises a cellular component, for example, a living or viable cellular component, and a filler component effectively providing volume and support for viability and growth of the cells and/or other tissue when the composition is injected into a target region of a patient.

The present compositions, when injected into a target region of a patient, provide relatively long term, for example, greater than six months or more, of increased tissue filling and volurnizing, relative to a substantially identical composition which does not include a cellular component. The present composition also provides the support, structure and space within the tissue to allow for growth and regeneration of tissue. The cellular component in the present composition also provides for the production or stimulation of cytokines and intrinsic stimulators of tissue growth and maintenance at the site of injection.

The cellular component of the present compositions preferably comprises adipose-derived progenitor cells, for example, adipose-derived stem cells. In some embodiments of the invention, methods are provided for filling and regenerating tissue using such cell and filler compositions which include autologous cells, for example, autologous, adipose-derived adult stem and/or progenitor cells. The cellular component of the compositions provide relatively long-term tissue regeneration when combined with the filler component after the composition has been injected or implanted into a patient.

The filler component of the present compositions generally comprises a biocompatible material that can provide substantially immediate or short term tissue filling, and preferably , an environment conducive to cell or tissue growth.

In some embodiments, the filler component is a material that absorbs water and expands once injected into the body, to provide space for cell growth to enhance tissue regeneration. The filler component may be a material selected from the group consisting of hyaluronic acid (HA), collagen, crosslinked hyaluronic acid/collagen, hydrogel and combinations thereof.

In one aspect of the invention, the filler component may further include one or more additional materials or agents which are components of a natural extracellular matrix or peptides, derivatives or analogs of integrin binding molecules, that are capable of optimizing implanted cell viability and/or sustaining cell growth for a relatively long term or sustained period of time after the injection or implantation of the composition.

The filler component may comprise, for example, a hydrogel material combined with one or more other beneficial materials, for example, integrin binding molecules, integrin binding derivatives or analogs thereof, or peptides or peptide analogs with the potential to bind to integrins on the injected cell population. Such materials may be selected based on their ability to bind to growth factor receptors to thereby stimulate cell growth, for example, the injected adiopose cells and/or the influx of intrinsie tissue progenitors or cytokines.

Suitable integrin binding proteins useful in the context of the present invention include, but are not limited to, collagen, elastin, laminin, vimentin, and non-protein cell binding components such as heparin sulfate or other materials.

In one embodiment of the invention, the filler component comprises a crosslinked hyaluronic acid (HA)-based composition, or a crosslinked HA and collagen based composition, such as described in Schroeder et al., U.S. Patent Application No. 12/247,175, filed on October 7, 2008, having common assignee herewith, the entire disclosure thereof being incorporated herein by this reference.

In some embodiments, the filler component comprises a thermo-sensitive polymer, for example, a sol-gel transforming hydrogel, for example, a sol-gel transforming polysaccharide hydrogel composition. Suitable sol-gel transforming hydrogels exhibit the properties of being in the state of a liquid (sol) at room temperature or below, but form a hydrogel which is sufficiently gel-fike to hold its shape, when at physiological temperature, or body temperature (37°C). Suitable sol-gel transforming materials include the natural biopolymcr chitosan and derivatives thereof. In combination with glycerol phosphate (GP-sodium salt), this cationic polyelectrolyte becomes thermosensitive in diluted acids and can undergo gelation around body temperature.

Exemplary thermogelling hydrophobic blocks useful in the filler components in accordance with the invention include, for example, but are not limited to poly(propylene oxide), poly(lactide-co-glycolic acid), poly(N-isoprapylacrylamide), poly(propylene fumarate), poly(caprolactone), poly(urethane) and poly(organophosphazene).

The compositions of the present invention are useful for tissue "sculpting", tissue replacement or regeneration, improving scar formation or reducing existing scar tissue or increasing tissue elasticity tissue regeneration after mastectomy or lumpectomy, and other clinical settings where volumizing and tissue regeneration are desirable. In some specific embodiments of the invention, the compositions are useful for treatment of damaged tissue, for example, radiation-induced tissue damage, steroid induced lipoatrophy or surgical or trauma induced soft tissue loss. For example, the compositions can be used as an effective means of delivering cells to an area of radiation-induced tissue damage in order to reduce scarring, replenish lost tissue progenitors, or generally improve overall patient outcome. The present compositions can further be advantageous as a dermal coating or injectable, for example, for use in treatment of existing scar tissue, or for wound covering and prevention of scar formation. Some of the present compositions are useful in the treatment of radiation-induced tissue damage.

The present invention further provides methods for filling tissue located at or near a previously implanted prosthetic implant, for example, but not limited to, a conventional breast implant. More specifically, in some embodiments, methods are provided for filling a soft tissue target region wherein the target region is a breast region of a human being. The method may include the step of placing a prosthetic implant in a patient and subsequently placing a composition comprising a combined sol-gel transforming component and cellular component adjacent the breast implant to "sculpt" or smooth the breast, for example, to reduce or eliminate depressions or other anomalies in the breast tissue near the implant.

In yet another aspect, the invention provides methods for preparing an injectable or implantable tissue filling and regenerating composition. One such method comprises providing a filler material and providing a cellular material and combining or mixing the filler material with the cellular material to produce a useful filling and regenerating composition.

For example, the step of providing the filler component includes producing a HA/collagen filler component. For example, the step includes contacting HA with a cross-linker to allow cross-linking of thc HA by the cross-linker, thereby forming a first composition, contacting the first composition with collagen to allow cross-linking of the collagen by the cross-linker, thereby forming a second composition, and contacting the second composition with a HA solution to allow cross-linking of the HA in the solution by the cross-linker, thereby producing an HA/collagen filler component. The next step includes combining or mixing the HA/collagen filler component with a cellular component including a preparation of living cells, thereby forming an injectable or implantable filling and regenerating composition.

In one especially advantageous embodiment, the step of providing a cellular component may include obtaining living cells, preferably including adult stem cells, to be mixed with the filler component. For example, the invention may include the steps of extracting tissue, for example, adipose tissue, from a patient to be treated with the filling and regenerating composition. The extracted tissue may be processed in a manner suitable for obtaining for example, substantially isolating, adult stem cells therefrom.

In one aspect of the invention, adipose derived stem cells form at least a portion of the cellular component of the injectable or implantable filling and regenerating composition. Adipose cells may be harvested from a patient by conventional liposuction or aspiration techniques. The removed fat tissue would be separated, for example, by centrifugation, to yield a tissue fluid layer, a fat cell layer and a layer of oils or lipids. The fat cell layer is harvested.

Centrifugation may be accomplished in a syringe, a bag like a "blood bag", or an automated centrifugation system such as available from Cytori Therapeutics.

Any suitable, conventional mechanism which can provide separation of cells from extracted tissue may be utilized within the scope of the invention. For example, in some embodiments of the invention, simple gravity sedimentation techniques may be used to provide separation of cells from unwanted or unneeded acellular components. It is further contemplated that purified or enriched cell products may also be used such as enriched mesenchymal stem cells or other stem or progenitor cell types capable of differentiation to mature tissue cells. These would include stem and progenitor cell lines.

It is advantageous to utilize cellular materials that are not cultured for any significant period of time. For example, in some embodiments of the invention, cellular material which is extracted from a patient can be used as a component of a filler product for the patient within the same day. In other embodiments of the invention, cellular material is cryopreserved and thawed for subsequent use a component of a filler product for injections at a later time. Cultured cells may be utilized by incubation in a nutrient media with or without growth factors that would support the viability and expansion of progenitor cells capable of forming fat, blood vessel cells, dermal cells or muscle cells. Processing is described above and generally cells would be utilized in a relatively short period of time that day.

More specifically, in some embodiments of the invention, cell material is stored or preserved, for example, using cryopreservation, prior to mixing with the filler component. Cryopreservation involves controlled freezing and storage of cellular material in liquid nitrogen or in freezers capable of reaching temperatures of -80° C or lower.

Prior to being mixed with the filler/volumizing component, the cryopreserved cellular material is rapidly thawed, for example, at a temperature of about 37° C. In some embodiments, centrifugation of the thawed cellular material may be needed to remove any cellular debris, lipid or acellular material prior to mixing the thawed cryopreserved cells with the filler/volumizer component.

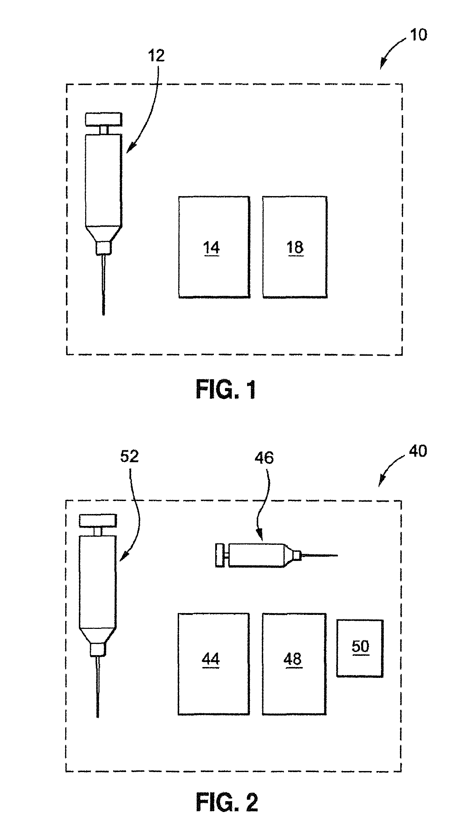

Turning now to Fig. 1, in a further aspect of the invention, a kit 10 for use in filling and regenerating tissue is provided. The kit 10 may comprise an injection device, for example, a syringe 12, an injectable composition 14 such as the cell/filler compositions for tissue filling and regeneration described elsewhere herein, and instructions for use 18. For example, composition 14 comprises a cellular component including living progenitor cells and the filler component comprises hyaluronic acid-based gel or a material that undergoes sol-gel transformation at physiological temperature.

Turning now to Fig. 2, in some embodiments, a kit 40 is provided which comprises a filler component 44, a suitable tool 46 for extracting cells from a region of a body of a patient, and instructions for use 48. The kit 40 may further comprise a mixing vessel 50 for combining cells extracted with the tool 46 with the filler component 44 to produce an injectable composition, for example a cell/filler mixture, such as described elsewhere herein. The kit 40 may further include an injection device, for example a syringe 52, for enabling injection of the cell/filler mixture into the patient, for example, in a different region, for example a breast region, of the body of the patient, wherein the different region would be benefited from both fill volume and tissue regeneration.

In some embodiments of the invention, compositions are provided as described elsewhere herein, the compositions being in the form of a topical coating, a wound covering, or similar form. In these embodiments, the compositions may comprise an element including a cellular component and a gel component, wherein the cellular component comprises cells mixed into or homogenized with the gel component. Advantageous uses of this embodiment includes, coating a wound or existing scar tissue to accelerate healing, prevent or reduce occurrence of scarring, or possibly reverse photo-, thermal- or radiation-induced tissue damage. Such a composition could also be injected around and/or under an existing scar to provide for regeneration of tissue and softening of the scarred region.

Applications of the present compositions, processes, methods and kits include their use in sculpting, tissue replacement or regeneration, improving scar formation, tissue regeneration after mastectomy or lumpectomy, and other clinical settings where volume and tissue regeneration are desirable. It is also contemplated that the present compositions can be used in conjunction with a prosthetic implant, for example a conventional breast implant, as a tissue filler, for example, to smooth or fill depressions and/or other areas of the body that may occur adjacent the prosthetic implant.

### Example

A composition useful for filling and regenerating soft tissue is prepared as follows:
Adipose tissue is collected using typical liposuction procedures. Lipoaspirate may be further separated by centrifugation to remove excess fluid or free lipid. Cellular material containing fat cells, endoothelial cells, mesymehymal cells and stem and progenitor cells would be collected and adjusted if necessary with a compatible solution to an appropriate cell concentration suitable for mixing with the filler component. Mixing could either be by agitation of the two components or by utilization of a bifurcated or dual chamber system that would mix the two components prior to or during the injection procedure. If a sol-gel transformation is involved with the filler component, a catalyst such as a salt or pH change would be achieved when the two components are mixed. If a thermal dependent sol-gel transformation is required for the filler, this may be achieved by an in vivo temperature shift post injection or by external manipulation of the temperature of the mixture prior to injection. Injection into the target site may be achieved using a needle or cannula attached to a syringe or delivery device.

While this invention has been described with respect to various specific examples and embodiments, it is to be understood that the invention is not limited thereto and that it can be variously practiced within the scope of the invention.

The present invention can also be summarised by the following embodiments (embs.):
Emb. 1. A process for augmenting soft tissue in a patient comprising the steps of:
   providing an injectable composition comprising a cellular component including living cells and a degradable filler component including a material conducive to maintaining viability of the cells;
   placing the composition in a target region of a patient where tissue volumizing and tissue regeneration is desirable.
Emb.2. The process of Emb.1 wherein the cellular component comprises stem cells.
Emb.3. The process of Emb.1 wherein the cellular component comprises cells selected from the group consisting of stem cell in-situ culture, progenitor cells, adipose-derived stem cells, endothelial cell precursors, proliferating cells, differentiation-committed cells, and regenerative cells.
Emb.4 The process of Emb.1 wherein the filler component comprises a component selected from the group consisting of hyaluronic acid, collagen, crosslinked hyaluronic acid/collagen, hydrogels and combinations thereof.
Emb.5. The process of Emb.1 wherein the filler component comprises a crosslinked hyaluronic acid having the characteristic of degrading in the target region at a rate about equal to a rate of tissue regeneration in the target region.
Emb.6 The process of Emb.1 wherein the target region is adjacent a prosthetic implant in the patient.
Emb.7. The process of Emb.1 wherein the target region is adjacent a breast implant in the patient.
Emb.8. A composition useful for filling and regenerating soft tissue, the composition being suitable for subcutaneous injection and comprising a cellular component including stem cells, and a filler component including at least one of hyaluronic acid-based gel and collagen.
Emb.9*.* The composition of Emb.8 wherein the filler component includes an integrin binding molecule or integrin binding derivative.
Emb.10. The composition of Emb.8 wherein the filler component includes at least one of an integrin binding protein or a peptide capable of promoting or maintaining implanted cell viability.
Emb.11. The composition of Emb.8 wherein the filler component comprises a sol-gel transforming polymer.
Emb.12. The composition of Emb.8 wherein the filler component comprises chitosan or a derivative thereof.
Emb.13. A process for augmenting soft tissue in a patient comprising the steps of:
   providing a composition comprising a cellular component and a sol-gel transforming component conducive to celt growth or viability;
   placing the composition in a target region of a patient where tissue volumizíng and tissue regeneration is desirable.
Emb.14. The process of Emb.13 wherein the step of placing comprising injecting the composition into the target region.
Emb.15. The process of Emb.13 wherein the sol-gel transforming component is a thermo-sensitive polymer.
Emb.16. The process of Emb.13 wherein the sol-gel transforming component includes chitosan or a derivative thereof.
Emb.17. A process for making a soft tissue filler comprising the steps of:
   providing a sol-gel transforming composition;
   providing a cellular component including living cells;
   combining the sol-gel transforming component with the cellular component when the sol-gel transforming component is a sol that gels at physiological temperature.
Emb.18. The process of Emb.17 wherein the living cells include stem cells.
Emb.19. The process of Emb.17 wherein the living cells include cells selected from the group consisting of stem cell in-situ culture, progenitor cells, adipose-derived stem cells, endothelial cell precursors, proliferating cells, differentiation-committed cells, and regenerative cells.
Emb.20. A method for filling soft tissue in a target region of a patient, the method comprising the steps of:
   providing a composition comprising a sol-gel transforming component and a cellular component including living cells, the sol-gel transforming component being in a liquid form; and
   placing the combined sol-gel transforming component and cellular component in a target region of a patient;
   wherein the sol component gels *in situ* and allows growth of the living cells in the patient.
Emb. 21. The method of Emb.20 wherein the target region is adjacent a prosthetic implant in the patient.
Emb.22. The method of Emb.20 wherein the target region is adjacent a breast implant in the patient.
Emb.23. A wound treatment composition comprising:
   an element comprising a gel component and a cellular component combined with the gel component and including living cells;
   the element being form of a dermal coating or injectable for covering or injecting existing scar tissue, wounds or other damaged tissue of a patient.
Emb.24. The composition according to Emb.23 wherein the gel component is a sol-gel transforming component in sol form when at less than physiological temperature.

## Claims

1. A composition useful for filling and regenerating soft tissue in a mammal, the composition being suitable for subcutaneous injection and comprising:
a cellular component including cells selected from the group consisting of stem cell in-situ culture, progenitor cells, adipose cells, adipose-derived stem cells, mesenchemal stem cells, endothelial cell precursors, proliferating cells, differentiation-committed cells, and regenerative cells and stem cell lines; and
a filler component selected from the group consisting of hyaluronic acid, chitosan, biodegradable polymers, collagen, crosslinked hyaluronic acid/collagen, hydrogels, derivatives and combinations thereof.

2. The composition of claim 1, wherein the cellular component includes stem cells and the filler component includes at least one of hyaluronic acid-based gel and collagen.

3. The composition of claim 1 or claim 2 wherein the filler component includes an integrin binding molecule or integrin binding derivative.

4. The composition of claim 1 or claim 2 wherein the filler component includes at least one of an integrin binding protein or a peptide capable of promoting or maintaining implanted cell viability.

5. The composition of claim 1 or claim 2 wherein the filler component comprises a sol-gel transforming polymer.

6. The composition of claim 1 or claim 2 wherein the filler component comprises chitosan or a derivative thereof.

7. A method of filling and regenerating soft tissue in a mammal using a composition according to any preceding claim, provided that the method is not therapeutic.

8. A composition according to any of claims 1 to 6 for use in filling and regenerating soft tissue in a mammal.

9. A wound treatment composition comprising:
an element comprising a gel component and a cellular component combined with the gel component and including living cells selected from the group consisting of stem cell in-situ culture, progenitor cells, adipose cells, adipose-derived stem cells, mesenchemal stem cells, endothelial cell precursors, proliferating cells, differentiation-committed cells, and regenerative cells and stem cell lines; and
the element being form of a dermal coating or injectable for covering or injecting existing scar tissue, wounds or other damaged tissue of a patient.

10. The composition according to claim 9 wherein the gel component is a sol-gel transforming component in sol form when at less than physiological temperature.

11. A composition according to claim 9 or claim 10 for use in would treatment in a mammal.
